# EUROPEAN PATENT APPLICATION

(11) **EP 4 699 602 A1**
(43) Date of publication of application: **25.02.2026**
(21) Application number: 25197349.1
(22) Date of filing: 21.08.2025
(51) Int. Cl.: A61K 9/48, A61K 31/445

(54) **A CAPSULE FORMULATION COMPRISING MIGALASTAT**

(30) Priority: 23.08.2024 TR 2024011147
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR); GOKSEN ERSOY, Elif, Istanbul (TR); OZTAS, Necla, Istanbul (TR)
(74) Representative: Genç Ilhan, Oznur

(57) **Abstract**

The present invention relates to a capsule formulation comprising migalastat hydrochloride and at least one lubricant, wherein the lubricant is sodium stearyl fumarate. The process is simple, rapid, cost effective, time-saving and industrially convenient process and is created to eliminate the disadvantages of active ingredient.

## Description

### Field of the Invention

The present invention relates to a capsule formulation comprising migalastat hydrochloride and at least one lubricant, wherein the lubricant is sodium stearyl fumarate. The process is simple, rapid, cost effective, time-saving and industrially convenient process and is created to eliminate the disadvantages of active ingredient.

### Background of the Invention

Fabry disease is a multi-systemic, X-linked lysosomal storage disease caused by decreased activity of alpha-galactosidase A and results in lysosomal accumulations of neutral glycosphingolipids and globotriaosylceramide GL-3. Angiokeratoma corporis diffusum is the typical skin lesion seen in Fabry disease and is linked to renal involvement, especially proteinuria.

Young adults presenting with a cerebrovascular event in association with myocardial infarction and renal dysfunction should be considered for Fabry disease. Abnormalities in almost any part of the body can be found with more predisposition of the skin, eye, kidney, heart, brain, and peripheral nervous system.

Migalastat was developed by Amicus therapeutics and granted approval in 2018 to treat fabry disease, a rare and progressive lysosomal storage disorder caused by mutations in the galactosidase alpha gene.

Migalastat was developed by Amicus therapeutics and granted approval in 2018 to treat fabry disease. Migalastat is an oral pharmacological chaperone of alpha-Gal A for the treatment of Fabry disease in adults who have amenable GLA variants. In these patients, migalastat works by stabilizing the body's dysfunctional alpha-Gal A enzyme so that it can clear the accumulation of glycosphingolipid disease substrate. Globally, it is estimated that approximately 35 to 50 percent of Fabry patients may have amenable GLA variants that are treatable with migalastat.

Migalastat, also known as 1-deoxy-galactonojirimycin or (2R,3S,4R,5S)-2-(hydroxymethyl)piperidine-3,4,5-triol is a compound having the following chemical formula I:

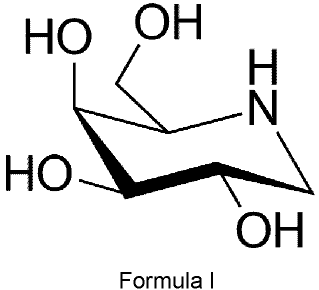

Migalastat is used in form of the hydrochloride, which is a white crystalline solid and is soluble in water. The molecule has four asymmetric carbon atoms with the same stereochemistry as the sugar D-galactose, but is missing the first hydroxyl group. It has a nitrogen atom in the ring instead of an oxygen, which makes it an iminosugar.

The patent EP3698792A1 disclose a pharmaceutical composition comprising migalastat hydrochloride, magnesium stearate and pregelatinized starch.

In the prior art formulation, magnesium stearate was used as lubricant. However, although magnesium stearate is a good lubricant, it is known to have some disadvantages. Magnesium stearate is practically insoluble in water and this hydrophobic property may delay capsule dissolution. Capsule dissolution is sensitive to both the amount of lubricant in the formulation and the blending time. In magnesium stearate, long blending times can result in the formulation of hydrophobic powder beds that do not disperse easily, and excessive blending can cause jamming problems. It can also form agglomerates on other capsule excipients during prolonged mixing, resulting in prolonged drug release time, reduced hardness and increased disintegration time.

Migalastat formulations are known in the prior art. However, there is still a need for a formulation that has the desired dissolution profile and provides a faster process.

### Detailed description of the Invention

The main object of the present invention is a capsule formulation comprising migalastat hydrochloride having the desired dissolution profile.

Another object of the present invention is to provide a preparation process of a capsule formulation comprising migalastat hydrochloride which is faster process.

Migalastat formulations are known in the prior art. However, since there is still a need for a formulation that provides the desired dissolution profile and a faster process, we prepared a new formulation using sodium stearyl fumarate instead of magnesium stearate. It is known that sodium stearyl fumarate is less hydrophobic and has less dissolution retarding effect. We have seen that when we used sodium stearyl fumarate, it provided the desired dissolution profile and helped faster process formation.

According to one embodiment of the invention, a capsule formulation comprises migalastat hydrochloride and at least one lubricant, wherein the lubricant is sodium stearyl fumarate.

According to one embodiment of the present invention, the amount of the migalastat hydrochloride is between 60.0% and 80.0% by weight of the total formulation. Preferably, the amount of migalastat hydrochloride is between 70.0% and 73.0% by weight of the total formulation.

According to one embodiment of the present invention, the amount of the lubricant is between 0.1% and 5.0% by weight of the total formulation.

According to this embodiment of the present invention, the capsule formulation further comprises at least one diluent.

Suitable diluents are selected from the group comprising pregelatinized starch, corn starch, microcrystalline cellulose, lactose monohydrate, lactose anhydrous, lactose anhydrate, dicalcium phosphate anhydrate, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate or a mixture thereof.

According to this embodiment of the present invention, the diluent is pregelatinized starch.

According to one embodiment of the present invention, the amount of the diluent is between 15.0% and 35.0% by weight of the total formulation.

As used herein, 'particle size' means the cumulative volume size distribution as tested by any conventionally accepted method such as the laser diffraction method (i.e. Malvern analysis). The term d (0.9) means the size at which %90 by volume of the particles are finer, the term d (0.5) means the size at which %50 by volume of the particles are finer, the term d (0.1) means the size at which %10 by volume of the particles are finer.

According to one embodiment, migalastat hydrochloride has a d (0.9) particle size between 1 µm and 24 µm or between 25 µm and 53 µm or between 55 µm and 87 µm or between 90 µm and 108 µm or between 110 µm and 147 µm or between 150 µm and 183 µm or between 185 µm and 200 µm.

According to this embodiment of the present invention, the capsule formulation comprises;
- Migalastat Hydrochloride
- A Diluent
- Sodium stearyl fumarate

According to this embodiment of the present invention, the capsule formulations comprises;
- Migalastat Hydrochloride
- Pregelatinized starch
- Sodium stearyl fumarate

According to this embodiment of the present invention, the capsule formulations comprises;
- 60.0% and 80.0% by weight of Migalastat Hydrochloride
- 0.1% and 5.0% by weight of Sodium stearyl fumarate

According to this embodiment of the invention, a method for preparing capsule formulations comprises the following steps:
- Mixing and sieving Migalastat hydrochloride and diluent,
- Adding sodium stearly fumarate to the mixture in step (a) and mixing,
- Filling the mixture into capsules.

### Example 1;

| **Ingredients** | **% by weight** | **% by weight** |
|---|---|---|
| Migalastat hydrochloride | 72.5 | 60-80 |
| A Diluent | 26.5 | 15-35 |
| Sodium Stearyl Fumarate | 1 | 0.1-5 |
| **Total Weight** | **100** | **100** |

### Example 2;

| **Ingredients** | **% by weight** | **% by weight** |
|---|---|---|
| Migalastat hydrochloride | 72.5 | 60-80 |
| Pregelatinized Starch | 26.5 | 15-35 |
| Sodium Stearyl Fumarate | 1 | 0.1-5 |
| **Total Weight** | **100** | **100** |

A process for example 2;
a) Mixing and sieving Migalastat hydrochloride and pregelatinized starch,
b) Adding sodium sterile fumarate to the mixture in step (a) and mixing,
c) Filling the mixture into capsules.

## Claims

1. A capsule formulation comprising migalastat hydrochloride and at least one lubricant, wherein the lubricant is sodium stearyl fumarate.

2. The capsule formulation according to claim 1, wherein the amount of the migalastat hydrochloride is between 60.0% and 80.0% by weight of the total formulation.

3. The capsule formulation according to claim 1, wherein the amount of the lubricant is between 0.1% and 5.0% by weight of the total formulation.

4. The capsule formulation according to claim 1, wherein the capsule formulation further comprises at least one diluent.

5. The capsule formulation according to claim 4, wherein diluents are selected from the group comprising pregelatinized starch, corn starch, microcrystalline cellulose, lactose monohydrate, lactose anhydrous, lactose anhydrate, dicalcium phosphate anhydrate, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate or a mixture thereof.

6. The capsule formulation according to claim 4 or 5, wherein the diluent is pregelatinized starch.

7. The capsule formulation according to claim 6, wherein the amount of the diluent is between 15.0% and 35.0% by weight of the total formulation.

8. The capsule formulation according to any preceding claim, wherein the capsule formulation comprises;
• Migalastat Hydrochloride
• A Diluent
• Sodium stearyl fumarate

9. The capsule formulation according to any preceding claim, wherein the capsule formulations comprises;
• Migalastat Hydrochloride
• Pregelatinized starch
• Sodium stearyl fumarate

10. The capsule formulation according to any preceding claim, wherein the capsule formulations comprises;
• 60.0% and 80.0% by weight of Migalastat Hydrochloride
• 0.1% and 5.0% by weight of Sodium stearyl fumarate

11. A method for preparing capsule formulations comprises the following steps:
a. Mixing and sieving Migalastat hydrochloride and diluent,
b. Adding sodium stearly fumarate to the mixture in step (a) and mixing,
c. Filling the mixture into capsules.
